# FASCICULE DE BREVET EUROPEEN

(11) **EP 4 632 645 B1**
(45) Date de publication et mention de la délivrance du brevet: **30.09.2026**
(21) Numéro de dépôt: 25169639.9
(22) Date de dépôt: 10.04.2025
(51) Int. Cl.: G06Q 10/0639, G06Q 50/40

(54) **PROCÉDÉ DE DÉTERMINATION D'UN NIVEAU DE FATIGUE OBJECTIF D'UN OPÉRATEUR EFFECTUANT UNE MISSION, SYSTÈME DE DÉTERMINATION ASSOCIÉ ET PROCÉDÉ D ESTIMATION ET DE DÉTERMINATION D'UN TEL NIVEAU DE FATIGUE**
VERFAHREN ZUR BESTIMMUNG EINES OBJEKTIVEN ERMÜDUNGSNIVEAUS EINES MISSIONSFÜHRENDEN BEDIENERS, ZUGEHÖRIGES BESTIMMUNGSSYSTEM UND VERFAHREN ZUR SCHÄTZUNG UND BESTIMMUNG SOLCH EINES ERMÜDUNGSNIVEAUS
METHOD FOR DETERMINING AN OBJECTIVE FATIGUE LEVEL OF AN OPERATOR PERFORMING A MISSION, ASSOCIATED DETERMINATION SYSTEM AND METHOD FOR ESTIMATING AND DETERMINING SUCH A FATIGUE LEVEL

(30) Priorité: 12.04.2024 FR 2403805
(43) Date de publication de la demande: 15.10.2025
(73) Titulaire: THALES, 92190 Meudon (FR)
(72) Inventeur: BERTHELOT, Bastien, 33700 MERIGNAC (FR); IBANEZ, Vincent, 33700 MERIGNAC (FR)
(74) Mandataire: Lavoix

(56) Documents cités:
- CN-A- 116 313 079
- FR-A1- 3 104 935
- US-A1- 2016 090 097
- BRAND YANNICK ET AL: "Model-based prediction of workload for adaptive associate systems", 2017 IEEE INTERNATIONAL CONFERENCE ON SYSTEMS, MAN, AND CYBERNETICS (SMC), IEEE, 5 October 2017 (2017-10-05), pages 1722 - 1727, XP033271193, DOI: 10.1109/SMC.2017.8122864
- SYLVIE SAGET ET AL: "Cooperative interface of a swarm of UAVs", ARXIV.ORG, CORNELL UNIVERSITY LIBRARY, 201 OLIN LIBRARY CORNELL UNIVERSITY ITHACA, NY 14853, 3 November 2008 (2008-11-03), XP080438960
- DELLRAGNOLA FABIO ET AL: "Machine-Learning Based Monitoring of Cognitive Workload in Rescue Missions With Drones", IEEE JOURNAL OF BIOMEDICAL AND HEALTH INFORMATICS, IEEE, PISCATAWAY, NJ, USA, vol. 26, no. 9, 25 June 2022 (2022-06-25), pages 4751 - 4762, XP011919437, ISSN: 2168-2194, [retrieved on 20220627], DOI: 10.1109/JBHI.2022.3186625

## Description

La présente invention concerne un procédé de détermination d'un niveau de fatigue objectif d'un opérateur effectuant une mission.

La présente invention concerne en outre un système de détermination mettant en œuvre un tel procédé de détermination.

La présente invention concerne également un procédé d'estimation et de détermination d'un tel niveau de fatigue.

L'invention se situe plus particulièrement dans le domaine technique de détermination de la fatigue d'un opérateur.

L'opérateur évolue par exemple dans un contexte opérationnel critique. Autrement dit, la fatigue de l'opérateur dans ce contexte critique peut conduire à des conséquences importantes. Cela est le cas notamment dans le domaine aéronautique, aérospatial, ferroviaire, nucléaire, de la médecine, etc.

Dans le domaine aéronautique, on connaît notamment des modèles biomathématiques de prédiction de fatigue. Ces modèles présentent des outils permettant de prédire les niveaux de fatigue des membres d'équipage. Les prédictions reposent sur une connaissance scientifique des facteurs générant de la fatigue et sur les informations de planification de la mission. Ces modèles sont utilisés en tant que solution pour prédire la fatigue et sont actuellement disponibles sur le marché.

Toutefois, les modèles biomathématiques de fatigue existants ne peuvent pas estimer un niveau de fatigue de façon précise et objective pour tout type de mission et pour tout opérateur. En effet, ces modèles biomathématiques sont généralement basés sur les niveaux de fatigue moyens et d'autres données issues des campagnes ponctuelles telles que des sondages ou des déclarations obtenues auprès d'un nombre limité d'individus. Or, ces sondages et déclarations s'appuient généralement sur des ressentis personnels des opérateurs pouvant être biaisés par des facteurs culturels, professionnels ou opérationnels. Ils ne correspondent pas à une façon objective d'estimer un niveau de fatigue.

En outre, les modèles biomathématiques existants s'appuient sur des principes génériques et ne reflètent pas toujours la réalité des opérations. Ils ne prennent pas en

compte certains facteurs opérationnels tels que la météo, la saison, ou encore le type de moyen de transport utilisé pour la mission.

Le document BRAND YANNICK ET AL: "Model-based prediction of workload for adaptive associate systems", 2017 IEEE INTERNATIONAL CONFERENCE ON SYSTEMS, MAN, AND CYBERNETICS (SMC), divulgue une estimation de fatigue basée sur un modèle de prédiction biomathématique.

Le document FR3104935A1 fait également partie de l'art antérieur.

Le but de l'invention est alors de proposer un moyen permettant de déterminer de façon objective et précise le niveau de fatigue d'un opérateur.

A cet effet, l'invention a pour objet un procédé de détermination d'un niveau de fatigue objectif d'un opérateur effectuant une mission, le procédé comprenant les étapes suivantes :
- acquisition d'une première estimation de fatigue déterminée par un modèle biomathématique de prédiction de fatigue à partir d'une pluralité de données de mission relatives à la mission ;
- acquisition d'une deuxième estimation de fatigue déterminée par un modèle physiologique de prédiction de fatigue à partir de données physiologiques de l'opérateur ; et
- détermination d'un niveau de fatigue objectif par fusion des deux estimations de fatigue.

Suivant d'autres aspects avantageux de l'invention, le procédé de détermination comprend une ou plusieurs des caractéristiques suivantes, prises isolément ou suivant toutes les combinaisons techniquement possibles :
- la fusion est effectuée par un recalage de la première estimation de fatigue par la deuxième estimation de fatigue ;
- le recalage est fait en utilisant un filtre optimal ;
- le filtre optimal est un filtre de Kalman et/ou un filtre particulaire ;
- la mise en 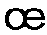 uvre du filtre optimal comprend les sous-étapes suivantes :
   + calcul d'une pondération initiale pour un instant T à partir de la première estimation de fatigue acquise pour un instant précédent T-1, la pondération initiale correspondant à l'incertitude de la première estimation de fatigue ;
   + prédiction d'une première estimation de fatigue théorique pour l'instant T à partir de la pondération initiale pour l'instant T, de la première estimation de fatigue acquise pour l'instant précédent T-1 et d'une fonction d'évolution théorique de la fatigue ;
   + modification de la pondération initiale à partir d'une probabilité conditionnelle de la deuxième estimation de fatigue acquise pour l'instant T sachant la première estimation de fatigue théorique pour l'instant T ; et
   + estimation d'un niveau de fatigue objectif pour l'instant T à partir de la pondération initiale pour l'instant T modifiée et la première estimation de fatigue théorique pour l'instant T ;
- la mise œuvre du filtre optimal comprend en outre une sous-étape de génération d'un ensemble de particules, chaque particule comportant une première estimation de fatigue acquise pour un instant précédent T-1 et une pondération initiale préalablement non définie associée à la première estimation de fatigue ;
   lesdites sous-étapes de calcul, de prédiction, de modification et d'estimation étant mises en œuvre pour chacune des particules ;
   le procédé comprenant en outre une sous-étape d'estimation d'un niveau de fatigue objectif résultant à partir d'une moyenne pondérée des estimations du niveau de fatigue pour les différentes particules.
- la fusion est effectuée par un algorithme d'apprentissage supervisé apte à extraire un ensemble supervisé de signature(s) lié(s) au niveau de fatigue objectif à partir des deux estimations de fatigue, un modèle de l'algorithme d'apprentissage supervisé étant choisi parmi le groupe consistant en : réseaux de neurones, régression logistique, machine à vecteurs de support, et méthode des k plus proches voisins ;
- la fusion est effectuée par un algorithme d'apprentissage non supervisé apte à extraire un ensemble non supervisé de signature(s) lié(s) au niveau de fatigue objectif à partir des deux estimations de fatigue, un modèle de l'algorithme d'apprentissage non supervisé étant choisi parmi le groupe consistant en : regroupement hiérarchique, partitionnement en K-moyennes, cartes auto-organisatrices, et mélange gaussiens ;
- le modèle biomathématique est construit à partir de données subjectives et/ou statistiques relatives à une pluralité d'individus.
- la mission de l'opérateur est le pilotage d'un aéronef et dans lequel la pluralité de données de mission comprennent au moins un type de données choisi dans le groupe comprenant :
   + données relatives au planning d'un équipage dont fait partie l'opérateur ;
   + données relatives à la configuration d'une compagnie aérienne.
- les données physiologiques comprennent au moins un type de données choisi dans le groupe comprenant :
   + des images de l'opérateur ;
   + un rythme cardiaque ;
   + une pression artérielle ;
   + une inspiration d'oxygène ;
   + une sudation ;
   + une saturation en oxygène ;
   + un taux de déshydratation ;
- les données physiologiques de l'opérateur sont mesurées pendant la mission.

L'invention concerne également un système de détermination d'un niveau de fatigue objectif comprenant des moyens techniques configurés pour mettre en œuvre le procédé selon l'une quelconque des caractéristiques décrites ci-dessus.

L'invention concerne également un procédé d'estimation et de détermination d'un niveau de fatigue d'un opérateur, comprenant les étapes suivantes :
- détermination d'une première estimation de fatigue déterminée par un modèle biomathématique de prédiction de fatigue à partir d'une pluralité de données de mission relatives à la mission ;
- détermination d'une deuxième estimation de fatigue déterminée par un modèle physiologique de prédiction de fatigue à partir des données physiologiques de l'opérateur ;
- mise en œuvre du procédé de détermination selon l'une quelconque des caractéristiques décrites ci-dessus.

L'invention apparaîtra plus clairement à la lecture de la description qui va suivre, donnée uniquement à titre d'exemple non limitatif, et faite en référence aux dessins dans lesquels :
la figure 1 est une représentation schématique d'une architecture d'estimation et de détermination d'un niveau de fatigue d'un opérateur, selon l'invention ;
la figure 2 est une représentation schématique d'un système de détermination d'un niveau de fatigue objectif, le système de détermination faisant partie de l'artichitecture d'estimation et de détermination de la figure 1 ; et
la figure 3 est un organigramme d'un procédé d'estimation et de détermination d'un niveau de fatigue, le procédé étant mis en œuvre par l'archiutecture de la figure 1.

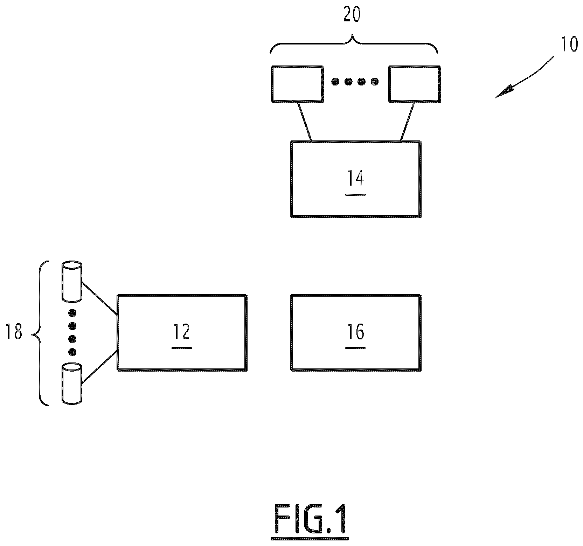

La figure 1 représente une architecture 10 d'estimation et de détermination d'un niveau de fatigue d'un opérateur. L'architetcure 10 permet d'estimer et de déterminer un niveau de fatigue d'un opérateur.

Avantageusement, l'architecture 10 est utilisable dans le domaine aéronautique. Dans un tel cas, l'opérateur fait partie du personnel navigant, notamment du personnel navigant commercial. Selon d'autres exemples, l'opérateur fait partie des opérateurs de planification de vols ou des opérateurs de maintenance ou des opérateurs de contrôle d'aéronef ou des contrôleurs aériens.

Avantageusement, l'opérateur est un pilote apte à piloter un aéronef.

Par aéronef, on entend tout engin volant pilotable à partir du cockpit de celui-ci, comme cela est le cas par exemple d'un avion ou d'un hélicoptère, ou alors à distance de celui-ci, comme cela est le cas par exemple d'un drone.

De manière générale, la notion de l'opérateur peut s'appliquer à toute autre personne effectuant une mission critique, par exemple dans le domaine de transport (ferroviaire ou poids lourd ou tout transport par exemple) ou dans le domaine nucléaire ou spatial, ou dans la médecine.

Comme indiqué précédemment, l'opérateur effectue une mission qui est déterminée par le domaine de son activité.

En particulier, la mission de l'opérateur comprend une pluralité de tâches définies selon les compétences de l'opérateur.

Lorsque l'opérateur est un pilote d'aéronef, sa mission consiste généralement à un pilotage de l'aéronef d'un point de départ à un point de sa destination.

L'architecture d'estimation et de détermination 10 comprend un premier système d'estimation de fatigue 12, un deuxième système d'estimation de fatigue 14 et un système de détermination objective de fatigue 16.

Le premier système d'estimation de fatigue 12 est configuré pour déterminer une première estimation de fatigue à partir des données de mission, en mettant en œuvre un modèle biomathématique de prédiction de fatigue.

Pour ce faire, le premier système d'estimation de fatigue 12 est connecté à une ou plusieurs bases de données 18 contenant des données de mission. Ce système 12 peut par exemple être connecté à cette ou ces base(s) de données 18 de manière directe ou indirecte, via par exemple un réseau informatique.

Les données de mission décrivent la mission qui doit être effectuée par l'opérateur.

Ces données de mission comprennent par exemple les horaires de la mission (i.e. début, fin, durée), le poste devant être occupé par l'opérateur pendant cette mission (pilote, co-pilote ou autre poste critique par exemple dans le nucléaire ou contrôle aérien), composition de l'équipe (par exemple le nombre de pilotes nécessaire pour le vol à effectuer), la position du jour de travail dans la période (c'est-à-dire le numéro de jour dans la séquence de travail), les informations sur la planification de la mission (mission planifiée, replanifiée et dans ce dernier cas, antériorité de cette replanification), plage de temps de la mission (matinale, en journée, en soirée, nocturne), type d'avion ou tout autre poste de travail sur lequel la mission sera effectuée.

Avantageusement, lorsque la mission de l'opérateur est le pilotage d'un aéronef, les de données de mission 18 comprennent au moins un type de données choisi dans le groupe comprenant :
- données relatives au planning d'un équipage dont fait partie l'opérateur ;
- données relatives à la configuration d'une compagnie aérienne.

Avantageusement, les données de mission sont issues de la compagnie aérienne pour laquelle l'opérateur effectue sa mission. Dans un tel cas, la ou les base(s) de données 18 sont avantageusement mises à disposition du premier système d'estimation 12 par la compagnie aérienne.

Le modèle biomathématique de prédiction de fatigue est par exemple un modèle connu en soi qui permet de déterminer un niveau de fatigue à partir des données de mission, telles que définies ci-dessus. Le modèle biomathématique est par exemple construit à partir de données subjectives et/ou statistiques relatives à une pluralité d'individus. Chacun de ces individus présente par exemple un opérateur effectuant une mission identique ou similaire à celle de l'opérateur pour lequel le niveau de fatigue est déterminé par l'architecture d'estimation et de détermination 10.

De manière avantageuse, les données utilisées pour construire le modèle biomathématique sont déterminées préalablement à partir de données subjectives et/ou statistiques relatives auxdits individus. Ces données sont par exemple collectées suite aux auto-déclarations de ces individus et/ou des campagnes ponctuelles de sondage. Ces données peuvent également être traitées statistiquement afin de les rendre applicables aux autres individus.

À titre d'exemple, un modèle biomathématique peut être construit ou ajusté à partir des données déclaratives par des pilotes à la suite de chaque vol effectué. Le pilote peut par exemple déclarer son niveau de fatigue subjective après chaque vol. Le modèle peut ainsi associer différentes durées de vol à des différents niveaux de fatigue ressentie par les pilotes. Ainsi, un tel modèle est capable de déterminer un niveau de fatigue ressentie pour un instant donné durant le vol.

Un modèle biomathématique simple peut prendre par exemple la forme des abaques associant une valeur à une autre. Un modèle biomathématique plus complexe peut prendre la forme d'une formule associant une valeur à une pluralité d'autres valeurs et comprenant des coefficients déterminés préalablement à partir de données subjectives et/ou statistiques relatives aux individus.

Le niveau de fatigue déterminé par le modèle biomathématique présente une valeur (par exemple un nombre ou un caractère) déterminée sur une échelle propre à ce modèle.

Le deuxième système d'estimation de fatigue 14 est configuré pour déterminer une deuxième estimation de fatigue à partir des données physiologiques de l'opérateur, en mettant en œuvre un modèle physiologique de prédiction de fatigue.

Les données physiologiques de l'opérateur présentent tout type de données permettant de caractériser l'état physique de l'opérateur. Ces données physiologiques sont avantageusement acquises lors de la mission ou alors juste avant la mission ou alors juste après la mission.

Avantageusement, les données physiologiques de l'opérateur comprennent au moins un type de données choisi dans le groupe comprenant :
- des images de l'opérateur ;
- un rythme cardiaque ;
- une pression artérielle ;
- une inspiration d'oxygène ;
- une fréquence de respiration ;
- une amplitude de respiration ;
- une sudation ;
- une saturation en oxygène ;
- un taux de déshydratation.

Pour acquérir les données physiologiques, le deuxième système d'estimation 14 est connecté directement ou indirectement à une pluralité de capteurs 20 disposés par exemple dans le poste de travail de l'opérateur. Par exemple, ces capteurs 20 sont disposés de manière fixe et/ou amovible dans le cockpit de l'aéronef piloté par l'opérateur. Ces capteurs 20 peuvent par exemple être connectés au deuxième système d'estimation 14 via un réseau informatique.

En particulier, la pluralité de capteurs 20 comprend tout capteur permettant d'acquérir les données physiologiques de l'opérateur.

Par exemple, la pluralité de capteurs 20 comprend une caméra (non représentée) configurée pour acquérir des images de l'opérateur et un capteur de rythme cardiaque (non représenté) permettant de mesurer le rythme cardiaque de l'opérateur.

La caméra est par exemple orientée vers l'opérateur ou présente des moyens permettant de l'orienter selon la position de l'opérateur.

Le capteur de rythme cardiaque de l'opérateur est configuré par exemple pour être positionné autour d'un poignet de l'opérateur.

À cet effet, le capteur de rythme cardiaque présente par exemple un bracelet susceptible d'être fixé sur le poignet de l'opérateur et une partie sensible qui est destinée à mesurer le rythme cardiaque de l'opérateur lorsque le bracelet est fixé sur son poignet.

La mesure du rythme cardiaque s'effectue par exemple par la partie sensible, par la technique appelée photopléthysmographie, dit PPG. Alternativement, la partie sensible est configurée pour effectuer la mesure du rythme cardiaque à partir d'une analyse de réponse électrique par le poignet de l'opérateur ou par analyse de signaux de radar se propageant dans le poignet de l'opérateur.

Dans certains exemples, le capteur de rythme cardiaque est configuré pour mesurer d'autres paramètres physiologiques de l'opérateur tels que la pression artérielle, l'inspiration de l'oxygène, la sudation, le taux de déshydratation, etc.

Pour la saturation en oxygène, le capteur de rythme cardiaque est par exemple configuré pour émettre en direction de la peau de l'opérateur et recevoir un signal lumineux comprenant au moins deux longueurs d'onde. Une première longueur d'onde correspondant à une longueur d'onde absorbée par les globules rouges saturés, une deuxième longueur d'onde correspondant à une longueur d'onde absorbée par les globules rouges non saturés. Pour déterminer la saturation en oxygène, le capteur de rythme cardiaque est alors configuré pour comparer l'intensité lumineuse reçue en réponse à chacune des deux longueurs d'onde.

De manière générale, le capteur de rythme cardiaque peut se présenter sous la forme d'une montre connectée pour par exemple mesurer son rythme cardiaque.

Bien entendu, les fonctionnalités précitées du capteur de rythme cardiaque peuvent former des capteurs séparés.

Le modèle physiologique de prédiction de fatigue est configuré pour déterminer un niveau de fatigue de l'opérateur en utilisant ses données physiologiques telles que décrites précédemment. Ce niveau de fatigue présente une valeur (par exemple un nombre ou un caractère) déterminée sur une échelle propre à ce modèle.

Pour déterminer le niveau de fatigue, le modèle physiologique analyse les données physiologiques selon des algorithmes prédéterminés et associe un niveau de fatigue conformément à cette analyse.

Par exemple, le modèle physiologique peut analyser les images de l'opérateur afin de déterminer le nombre de clignotements des yeux, la fréquence de bâillement, et/ou tout autre geste susceptible d'être causé par la fatigue. Le modèle physiologique peut analyser par exemple le rythme cardiaque de l'opérateur et le coupler aux informations relatives à ses gestes.

Chacun des systèmes d'estimation 12, 14 décrits ci-dessus présente par exemple au moins partiellement une composante matérielle (telle qu'un circuit logique programmable de type FPGA) et/ou une composante logicielle. Dans ce dernier cas, la composante logicielle est par exemple mise en œuvre par un ordinateur comprenant au moins un processeur et une mémoire vive. Le modèle correspondant est par exemple stocké dans une mémoire non-volatile de cet ordinateur.

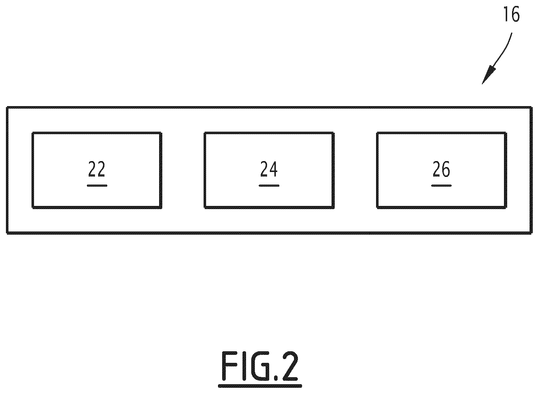

La figure 2 illustre en détail le système de détermination objective de fatigue 16. Ce système 16 est configuré pour fusionner la première et la deuxième estimations de fatigue afin de déterminer un niveau de fatigue objectif. Pour ce faire, le système de détermination objective de fatigue 16 comprend un module d'acquisition 22, un module de traitement 24 et un module de sortie 26.

Le module d'acquisition 22 est configuré pour acquérir les données en provenance des premier et deuxième systèmes d'estimation de fatigue 12, 14. En d'autres termes, le module d'acquisition 22 est configuré pour acquérir la première estimation de fatigue en provenance du premier système d'estimation de fatigue 12 et la deuxième estimation de fatigue en provenance du deuxième système d'estiamtion de fatigue 14.

Le module de traitement 24 est connecté en sortie du module d'acquisition 22 et est configuré pour déterminer un niveau de fatigue objectif par fusion de la première estimation de fatigue et de la deuxième estimation de fatigue, comme cela sera expliqué plus en détail par la suite.

En complément facultatif, le module de traitement 24 est en outre apte à identifier un ensemble de facteur(s) de risque associés au niveau de fatigue objectif.

Le module de sortie 26 est connecté en sortie du module de traitement 24. Ce module de sortie 26 est apte à transmettre le niveau de fatigue objectif à un système extérieur à l'architecture 10.

Chacun des modules 22, 24, 26 du système de détermination objective de fatigue 16 décrits ci-dessus présente par exemple au moins partiellement une composante matérielle (telle qu'un circuit logique programmable de type FPGA) et/ou une composante logicielle. Dans ce dernier cas, la composante logicielle est par exemple mise en œuvre par un ordinateur comprenant au moins un processeur et une mémoire vive.

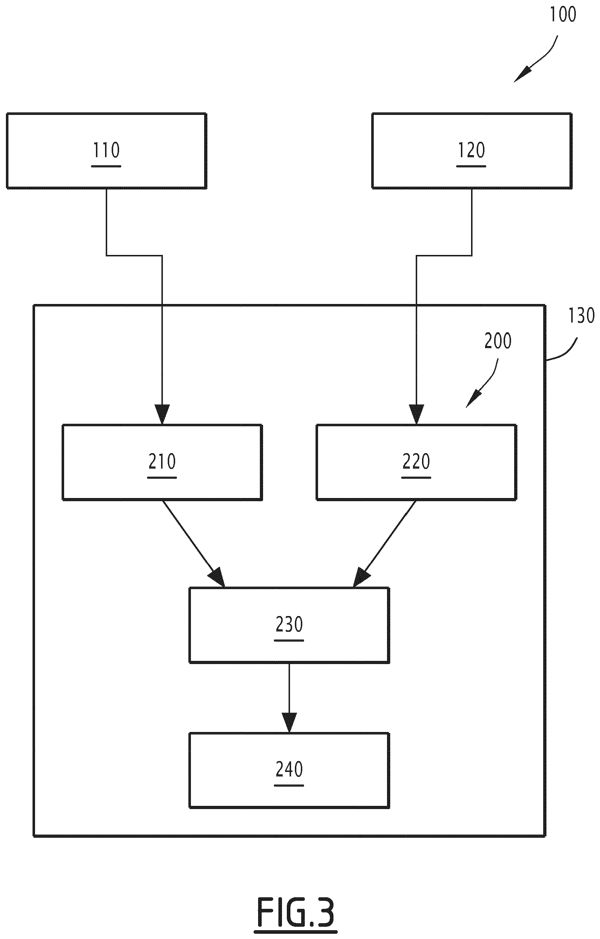

Le fonctionnement de l'architecture d'estimation et de détermination 10 selon l'invention va être à présent décrit en regard de la figure 3 représentant un organigramme du procédé 100, selon l'invention, d'estimation et de détermination d'un niveau de fatigue de l'opérateur. Ledit procédé est mis en œuvre par l'architecture 10.

Lors de l'étape 110 de ce procédé d'estimation et de détermination 100, le premier système d'estimation de fatigue 12 détermine une première estimation correspondant au niveau de fatigue déterminé par le modèle biomathématique, tel que décrit précédemment.

Ce niveau de fatigue est déterminé en utilisant les données de mission relatives à la mission de l'opérateur.

Cette étape 110 est par exemple mise en œuvre avant la mission de l'opérateur. Dans certains modes de réalisation, cette étape 110 peut être également mise en œuvre pendant la mission, par exemple tout au long de la mission. Dans un tel cas, la première estimation peut présenter une suite de données générées à différents instants par le modèle biomathématique.

Lors de l'étape 120 de ce procédé d'estimation et de détermination 100, le deuxième système d'estimation de fatigue 14 détermine une deuxième estimation correspondant au niveau de fatigue déterminé par le modèle physiologique, tel que décrit précédemment. L'étape 120 peut être mise en œuvre à la suite de l'étape 110 ou en parallèle avec cette étape 120.

Cette étape 120 est par exemple mise en œuvre pendant la mission de l'opérateur en utilisant les données physiologiques acquises également pendant la mission. Par exemple, cette étape mise en œuvre de manière continue tout au long de la mission de l'opérateur. Ainsi, la deuxième estimation peut présenter une suite de données générées à différents instants par le modèle physiologique.

Lors de l'étape 130 de ce procédé d'estimation et de détermination 100, le système de détermination objective de fatigue 16 met en œuvre un procédé de détermination 200 du niveau de fatigue objectif. Ce procédé 200 se base sur les deux estimations déterminées lors des étapes 110 et 120. Lors de l'étape 210 de ce procédé de détermination 200, le système de détermination objective de fatigue 16 acquiert, via son module d'acquisition 22, la première estimation de fatigue déterminée lors de l'étape 110.

Lors de l'étape 220 qui peut être mise en œuvre à la suite de l'étape 210 ou en parallèle avec cette étape 220, le module d'acquisition 22 acquiert la deuxième estimation déterminée lors de l'étape 120.

Lors de l'étape 230 suivante, le module de traitement 24 traite les estimations reçues afin de déterminer un niveau de fatigue objectif.

Pour cela, selon certains modes de réalisation, le module de traitement 24 peut tout d'abord normaliser les estimations reçues. Cela peut être par exemple le cas lorsque les modèles biomathématique et physiologique n'utilisent pas la même échelle de détermination de la fatigue.

Puis, le module de traitement 24 fusionne les estimations reçues.

Cette fusion est par exemple effectuée par recalage de la première estimation de fatigue par la deuxième estimation de fatigue.

De préférence, le recalage est fait en utilisant un filtre optimal tel qu'un filtre de Kalman ou un filtre particulaire.

Selon un exemple de réalisation, le filtre optimal est un filtre de Kalman.

Le filtre de Kalman calcule tout d'abord une pondération initiale pour un instant T à partir de la première estimation de fatigue acquise pour un instant précédent T-1. La pondération initiale correspond à l'incertitude de la première estimation de fatigue.

Ensuite, le filtre de Kalman prédit une première estimation de fatigue théorique pour l'instant T à partir de la pondération initiale pour l'instant T, de la première estimation de

fatigue acquise pour l'instant précédent T-1 et d'une fonction d'évolution théorique de la fatigue.

La fonction d'évolution théorique de la fatigue sert à estimer l'évolution de la première estimation de fatigue au cours du temps. Par exemple, la fonction d'évolution théorique de la fatigue correspond à une fonction linéaire par morceaux selon les phases de sommeil ou d'éveil de l'opérateur.

En variante, la fonction d'évolution théorique de la fatigue du filtre de Kalman correspond à une fonction plus complexe dépendant des données relatives au planning d'un équipage dont fait partie l'opérateur telles que la durée de la mission, ou encore le temps de la mission effectuée durant la nuit.

Le modèle d'évolution théorique de la fatigue du filtre de Kalman est par exemple modélisé selon l'équation suivante : ${x}_{bio , T} = f \left({x}_{bio , T - 1}\right) + {w}_{T}$ où
*x*_{*bio,T*-1} correspond à la première estimation de fatigue à l'instant précédent T-1 ;
*x_{bio,T}* correspond à la première estimation de fatigue théorique à l'instant T ;
*f* correspond à la fonction d'évolution théorique de la fatigue ; et
*w_{T}* correspond à la pondération initiale à l'instant T.

Le filtre de Kalman modifie par la suite la pondération initiale à partir d'une probabilité conditionnelle de la deuxième estimation de fatigue acquise pour l'instant précédent T-1 sachant la première estimation de fatigue théorique pour l'instant précédent T-1.

Enfin, le filtre de Kalman estime un niveau de fatigue objectif pour l'instant T à partir de la pondération initiale pour l'instant T modifiée et la première estimation de fatigue théorique pour l'instant T.

Alternativement, le filtre optimal est un filtre particulaire.

Le procédé avec le filtre particulaire génère tout d'abord un ensemble de particules. Chaque particule comporte une première estimation de fatigue acquise pour un instant précédent T-1 et une pondération initiale préalablement non définie associée à la première estimation de fatigue.

Le procédé avec le filtre particulaire présente des étapes en partie similaires au filtre de Kalman. Ainsi, le procédé avec le filtre particulaire met en œuvre les étapes de calcul, de prédiction et de modification du filtre de Kalman telles que décrites précédemment.

Suite à la modification, le procédé avec le filtre particulaire effectue, par exemple, l'étape de redimensionnement de l'ensemble de particules en gardant certaines particules en fonction de leurs pondérations initiales modifiées. Le redimensionnement permet, par exemple, de former un ensemble de particules modifié avec moins de particules que l'ensemble de particules et où les particules avec les pondérations les plus élevées sont plus susceptibles d'être choisies.

Les étapes de calcul, de prédiction, de modification et d'estimation sont mises en œuvre pour chacune des particules.

Dans un tel cas, la mise en œuvre du filtre optimal comprend en outre une sous-étape d'estimation d'un niveau de fatigue objectif résultant à partir d'une moyenne pondérée des estimations du niveau de fatigue pour les différentes particules. C'est donc ce niveau de fatigue objectif résultant qui est considéré par la suite comme niveau de fatigue objectif déterminé par le procédé de détermination 200.

En variante, la fusion est effectuée par un apprentissage automatique sur les données utilisées par le modèle biomathématique 12 appliquées sur des nouveaux cas du système de prédiction de fatigue 14.

De préférence, la fusion est effectuée par un algorithme d'apprentissage supervisé apte à extraire un ensemble supervisé de signature(s) lié(s) au niveau de fatigue objectif à partir des deux estimations de fatigue, un modèle de l'algorithme d'apprentissage supervisé étant choisi parmi le groupe consistant en : réseaux de neurones, régression logistique, machine à vecteurs de support, et méthode des k plus proches voisins.

Par exemple, la méthode d'apprentissage supervisée est un apprentissage symbolique à base de connaissances initiales du modèle biomathématique. Les connaissances initiales sont en constante évolution puisque des nouvelles règles peuvent être ajoutées aux connaissances initiales tout en maintenant une cohérence avec les connaissances initiales et en généralisant les connaissances apprises. Par exemple, le type d'apprentissage symbolique est un apprentissage par détection de similarités à partir d'exemples et contre-exemples. Par exemple, le type d'apprentissage symbolique est un apprentissage par recherche d'explications à partir d'exemples et contre-exemples.

Selon un autre exemple, la méthode d'apprentissage est un apprentissage numérique. Par exemple, le type d'apprentissage numérique est effectué via un ou plusieurs modèles statistiques.

En variante, la fusion est effectuée par un algorithme d'apprentissage non supervisé apte à extraire un ensemble non supervisé de signature(s) lié(s) au niveau de fatigue objectif à partir des deux estimations de fatigue, un modèle de l'algorithme d'apprentissage non supervisé étant choisi parmi le groupe consistant en : regroupement hiérarchique, partitionnement en K-moyennes, cartes auto-organisatrices, et mélange gaussiens.

Selon certains exemples, le procédé de détermination 200 comprend en outre une étape optionnelle lors de laquelle le module de traitement 24 identifie un ensemble de facteur(s) de risque associés au niveau de fatigue objectif.

Lors de l'étape 240 du procédé de détermination 200, le module de traitement 24 transmet au module de sortie 26 le niveau de fatigue objectif déterminé, accompagné éventuellement des facteurs de risque associés.

Le module de sortie 26 transmet ensuite ces données à tout système intéressé. Un tel système peut par exemple comprendre un moyen d'affichage, une base de données et/ou un serveur distant. Dans certains exemples, le niveau de fatigue objectif et éventuellement les facteurs de risque peuvent également être communiqués à l'opérateur.

On conçoit alors que la présente invention présente un certain nombre d'avantages. En particulier, l'invention permet de déterminer objectivement et précisément le niveau de fatigue objectif. Ainsi, indépendamment de la base ou les bases de données utilisées pour déterminer une première estimation de fatigue et indépendamment des capteurs 20 utilisés pour déterminer une deuxième estimation de fatigue, l'invention permet de déterminer un niveau de fatigue objectif qui est normalisé et donc comparable parmi plusieurs opérateurs et permettant d'identifier par la suite l'ensemble de facteurs de risque associés au niveau de fatigue objectif.

L'apprentissage supervisé permet d'améliorer encore plus la précision le niveau de fatigue objectif en raison d'une expérience considérable fusionnant les deux estimations.

L'apprentissage non supervisé permet d'améliorer la vitesse de calcul fusionnant les deux estimations.

L'utilisation d'un filtre optimal permet en outre d'avoir un contrôle sur la fusion des estimations. Lorsque le filtre optimal est un filtre particulaire, le niveau de fatigue objectif déterminé est particulièrement précis. Le filtre de Kalman permet d'obtenir une bonne approximation plus rapidement en simplifiant les étapes de calcul.

## Revendications

1. Procédé de détermination (200) d'un niveau de fatigue objectif d'un opérateur effectuant une mission, le procédé comprenant les étapes suivantes :
- acquisition (210) d'une première estimation de fatigue déterminée par un modèle biomathématique de prédiction de fatigue à partir d'une pluralité de données de mission relatives à la mission ;
- acquisition (220) d'une deuxième estimation de fatigue déterminée par un modèle physiologique de prédiction de fatigue à partir de données physiologiques de l'opérateur ; et
- détermination (230) d'un niveau de fatigue objectif par fusion des deux estimations de fatigue.

2. Procédé (200) selon la revendication 1, dans lequel la fusion est effectuée par un recalage de la première estimation de fatigue par la deuxième estimation de fatigue.

3. Procédé (200) selon la revendication 2, dans lequel le recalage est fait en utilisant un filtre optimal.

4. Procédé (200) selon la revendication 3, dans lequel le filtre optimal est un filtre de Kalman et/ou un filtre particulaire.

5. Procédé (200) selon la revendication 4, dans lequel la mise en œuvre du filtre optimal comprend les sous-étapes suivantes :
- calcul d'une pondération initiale pour un instant T à partir de la première estimation de fatigue acquise pour un instant précédent T-1, la pondération initiale correspondant à l'incertitude de la première estimation de fatigue ;
- prédiction d'une première estimation de fatigue théorique pour l'instant T à partir de la pondération initiale pour l'instant T, de la première estimation de fatigue acquise pour l'instant précédent T-1 et d'une fonction d'évolution théorique de la fatigue ;
- modification de la pondération initiale à partir d'une probabilité conditionnelle de la deuxième estimation de fatigue acquise pour l'instant T sachant la première estimation de fatigue théorique pour l'instant T ; et
- estimation d'un niveau de fatigue objectif pour l'instant T à partir de la pondération initiale pour l'instant T modifiée et la première estimation de fatigue théorique pour l'instant T.

6. Procédé (200) selon la revendication 5, dans lequel la mise œuvre du filtre optimal comprend en outre une sous-étape de génération d'un ensemble de particules, chaque particule comportant une première estimation de fatigue acquise pour un instant précédent T-1 et une pondération initiale préalablement non définie associée à la première estimation de fatigue ;
lesdites sous-étapes de calcul, de prédiction, de modification et d'estimation étant mises en œuvre pour chacune des particules ;
la mise œuvre du filtre optimal comprenant en outre une sous-étape d'estimation d'un niveau de fatigue objectif résultant à partir d'une moyenne pondérée des estimations du niveau de fatigue pour les différentes particules.

7. Procédé (200) selon la revendication 1, dans lequel la fusion est effectuée par un algorithme d'apprentissage supervisé apte à extraire un ensemble supervisé de signature(s) lié(s) au niveau de fatigue objectif à partir des deux estimations de fatigue, un modèle de l'algorithme d'apprentissage supervisé étant choisi parmi le groupe consistant en : réseaux de neurones, régression logistique, machine à vecteurs de support, et méthode des k plus proches voisins.

8. Procédé (200) selon la revendication 1, dans lequel la fusion est effectuée par un algorithme d'apprentissage non supervisé apte à extraire un ensemble non supervisé de signature(s) lié(s) au niveau de fatigue objectif à partir des deux estimations de fatigue, un modèle de l'algorithme d'apprentissage non supervisé étant choisi parmi le groupe consistant en : regroupement hiérarchique, partitionnement en K-moyennes, cartes auto-organisatrices, et mélange gaussiens.

9. Procédé (200) selon l'une quelconque des revendications précédentes, dans lequel le modèle biomathématique est construit à partir de données subjectives et/ou statistiques relatives à une pluralité d'individus.

10. Procédé (200) selon l'une quelconque des revendications précédentes, dans lequel la mission de l'opérateur est le pilotage d'un aéronef et dans lequel la pluralité de données de mission comprennent au moins un type de données choisi dans le groupe comprenant :
- données relatives au planning d'un équipage dont fait partie l'opérateur ;
- données relatives à la configuration d'une compagnie aérienne.

11. Procédé (200) selon l'une quelconque des revendications précédentes, dans lequel les données physiologiques comprennent au moins un type de données choisi dans le groupe comprenant :
- des images de l'opérateur ;
- un rythme cardiaque ;
- une pression artérielle ;
- une inspiration d'oxygène ;
- une sudation ;
- une saturation en oxygène ;
- un taux de déshydratation.

12. Procédé (200) selon l'une quelconque des revendications précédentes, dans lequel les données physiologiques de l'opérateur sont mesurées pendant la mission.

13. Système de détermination (16) d'un niveau de fatigue objectif comprenant des moyens techniques configurés pour mettre en 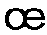 uvre le procédé selon l'une quelconque des revendications précédentes.

14. Procédé d'estimation et de détermination (100) d'un niveau de fatigue d'un opérateur, comprenant les étapes suivantes :
- détermination (110) d'une première estimation de fatigue déterminée par un modèle biomathématique de prédiction de fatigue à partir d'une pluralité de données de mission relatives à la mission ;
- détermination (120) d'une deuxième estimation de fatigue déterminée par un modèle physiologique de prédiction de fatigue à partir des données physiologiques de l'opérateur ;
- mise (130) en 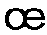 uvre du procédé de détermination (200) selon l'une des revendications 1 à 12.

## Patentansprüche

1. Verfahren zur Bestimmung (200) eines objektiven Ermüdungsniveaus eines Operators, der eine Mission ausführt, wobei das Verfahren die folgenden Schritte umfasst:
- Erfassen (210) einer ersten Ermüdungsschätzung, die durch ein biomathematisches Modell zur Vorhersage der Ermüdung ausgehend von einer Vielzahl von Missionsdaten bezüglich der Mission bestimmt wird;
- Erfassen (220) einer zweiten Ermüdungsschätzung, die durch ein physiologisches Modell zur Vorhersage der Ermüdung ausgehend von physiologischen Daten des Operators bestimmt wird; und
- Bestimmung (230) eines objektiven Ermüdungsniveaus durch Fusion der zwei Ermüdungsschätzungen.

2. Verfahren (200) nach Anspruch 1, wobei die Fusion durch eine Anpassung der ersten Ermüdungsschätzung durch die zweite Ermüdungsschätzung durchgeführt wird.

3. Verfahren (200) nach Anspruch 2, wobei die Anpassung unter Verwendung eines optimalen Filters erfolgt.

4. Verfahren (200) nach Anspruch 3, wobei der optimale Filter ein Kalman-Filter und/oder ein Partikelfilter ist.

5. Verfahren (200) nach Anspruch 4, wobei die Implementierung des optimalen Filters die folgenden Teilschritte umfasst:
- Berechnung einer anfänglichen Gewichtung für einen Zeitpunkt T ausgehend von der ersten Ermüdungsschätzung, die für einen vorhergehenden Zeitpunkt T-1 erfasst wurde, wobei die anfängliche Gewichtung der Unsicherheit der ersten Ermüdungsschätzung entspricht;
- Vorhersage einer theoretischen ersten Ermüdungsschätzung für den Zeitpunkt T ausgehend von der anfänglichen Gewichtung für den Zeitpunkt T, von der ersten Ermüdungsschätzung, die für den vorhergehenden Zeitpunkt T-1 erfasst wurde, und von einer theoretischen Entwicklungsfunktion der Ermüdung;
- Änderung der anfänglichen Gewichtung ausgehend von einer bedingten Wahrscheinlichkeit der zweiten Ermüdungsschätzung, die für den Zeitpunkt T erfasst wurde, unter der Bedingung der theoretischen ersten Ermüdungsschätzung für den Zeitpunkt T; und
- Schätzung eines objektiven Ermüdungsniveaus für den Zeitpunkt T ausgehend von der geänderten anfänglichen Gewichtung für den Zeitpunkt T und der theoretischen ersten Ermüdungsschätzung für den Zeitpunkt T.

6. Verfahren (200) nach Anspruch 5, wobei die Umsetzung des optimalen Filters ferner einen Teilschritt des Erzeugens einer Menge von Partikeln umfasst, wobei jedes Partikel eine erste Ermüdungsschätzung, die für einen vorhergehenden Zeitpunkt T-1 erfasst wurde, und eine zuvor nicht definierte anfängliche Gewichtung, die der ersten Ermüdungsschätzung zugeordnet ist, umfasst;
wobei die Teilschritte der Berechnung, der Vorhersage, der Änderung und der Schätzung für jedes der Partikel umgesetzt werden;
wobei die Umsetzung des optimalen Filters ferner einen Teilschritt der Schätzung eines resultierenden objektiven Ermüdungsniveaus ausgehend von einem gewichteten Mittelwert der Schätzungen des Ermüdungsniveaus für die verschiedenen Partikel umfasst.

7. Verfahren (200) nach Anspruch 1, wobei die Fusion durch einen überwachten Lernalgorithmus durchgeführt wird, der geeignet ist, eine überwachte Menge von Signatur(en), die mit dem objektiven Ermüdungsniveau verbunden ist/sind, ausgehend von den zwei Ermüdungsschätzungen zu extrahieren, wobei ein Modell des überwachten Lernalgorithmus aus der Gruppe ausgewählt ist, bestehend aus: neuronalen Netzen, logistischer Regression, Support-Vector-Maschine und Methode der k nächsten Nachbarn.

8. Verfahren (200) nach Anspruch 1, wobei die Fusion durch einen unüberwachten Lernalgorithmus durchgeführt wird, der geeignet ist, eine unüberwachte Menge von Signatur(en), die mit dem objektiven Ermüdungsniveau verbunden ist/sind, ausgehend von den zwei Ermüdungsschätzungen zu extrahieren, wobei ein Modell des unüberwachten Lernalgorithmus aus der Gruppe ausgewählt ist, bestehend aus: hierarchischer Gruppierung, K-Means-Partitionierung, selbstorganisierenden Karten und gaußschen Mischungen.

9. Verfahren (200) nach einem der vorhergehenden Ansprüche, wobei das biomathematische Modell ausgehend von subjektiven und/oder statistischen Daten bezüglich einer Vielzahl von Individuen konstruiert wird.

10. Verfahren (200) nach einem der vorhergehenden Ansprüche, wobei die Mission des Operators das Steuern eines Luftfahrzeugs ist und wobei die Vielzahl von Missionsdaten mindestens einen Datentyp umfasst, der aus der Gruppe ausgewählt ist, umfassend:
- Daten bezüglich der Planung einer Besatzung, zu der der Operator gehört;
- Daten bezüglich der Konfiguration einer Fluggesellschaft.

11. Verfahren (200) nach einem der vorhergehenden Ansprüche, wobei die physiologischen Daten mindestens einen Datentyp umfassen, der aus der Gruppe ausgewählt ist, umfassend:
- Bilder des Operators;
- eine Herzfrequenz;
- einen Blutdruck;
- eine Sauerstoffeinatmung;
- eine Schweißbildung;
- eine Sauerstoffsättigung;
- eine Dehydratationsrate.

12. Verfahren (200) nach einem der vorhergehenden Ansprüche, wobei die physiologischen Daten des Operators während der Mission gemessen werden.

13. System zur Bestimmung (16) eines objektiven Ermüdungsniveaus, umfassend technische Mittel, die konfiguriert sind, um das Verfahren nach einem der vorhergehenden Ansprüche umzusetzen.

14. Verfahren zur Schätzung und Bestimmung (100) eines Ermüdungsniveaus eines Operators, umfassend die folgenden Schritte:
- Bestimmung (110) einer ersten Ermüdungsschätzung, die durch ein biomathematisches Modell zur Vorhersage der Ermüdung aus einer Vielzahl von Missionsdaten bezüglich der Mission bestimmt wird;
- Bestimmung (120) einer zweiten Ermüdungsschätzung, die durch ein physiologisches Modell zur Vorhersage der Ermüdung aus den physiologischen Daten des Operators bestimmt wird;
- Durchführung (130) des Verfahrens zur Bestimmung (200) nach einem der Ansprüche 1 bis 12.

## Claims

1. A method of determining (200) an objective level of fatigue of an operator performing a mission, the method comprising the following steps:
- acquisition (210) of a first estimation of fatigue determined by a biomathematics model of prediction of fatigue from a plurality of mission data related to the mission;
- acquisition (220) of a second estimation of fatigue determined by a physiological model of prediction of fatigue from physiological data of the operator; and
- determination (230) of an objective level of fatigue by merging the two estimations of fatigue.

2. The method (200) according to claim 1, wherein the merging is performed by the fitting of the first estimation of fatigue using the second estimation of fatigue.

3. The method (200) according to claim 2, wherein the fitting is done using an optimal filter.

4. The method (200) according to claim 3, wherein the optimal filter is a Kalman filter and/or a particle filter.

5. The method (200) according to claim 4, wherein the implementation of the optimal filter comprises the following sub-steps:
- calculation of an initial weight for a time T from the first estimation of fatigue acquired for a preceding time T-1, the initial weight corresponding to the uncertainty of the first estimation of fatigue;
- prediction of a first theoretical estimation of fatigue for time T from the initial weighting for time T, the first estimation of fatigue acquired for the preceding time T-1 and a theoretical evolution of fatigue function;
- modification of the initial weight from a conditional probability of the second estimation of fatigue acquired for time T knowing the first estimation of theoretical fatigue for time T; and
- estimation of an objective level of fatigue for time T from the initial weighting for time T modified and the first estimation of theoretical fatigue for time T;

6. The method (200) according to claim 5, wherein the implementation of the optimal filter further comprises a sub-step of generation a set of particles, each particle comprising a first estimation of fatigue acquired for a preceding time T-1 and a priorly undefined initial weighting associated with the first estimation of fatigue;
said calculation, prediction, modification and estimation sub-steps being implemented for each of the particles;
the implementation of the optimal filter further comprises a sub-step of estimation of an objective level of fatigue resulting from a weighted average of the estimations of the level of fatigue for the different particles.

7. The method (200) according to claim 1, wherein the merging is performed by a supervised learning algorithm apt to extract a supervised set of signature(s) related to the objective level of fatigue from the two estimations of fatigue, a model of the supervised learning algorithm being chosen from the group consisting of: neural networks, logistic regression, support vector machine, and k-nearest neighbor.

8. The method (200) according to claim 1, wherein the merging is performed by an unsupervised learning algorithm apt to extract an unsupervised set of signature(s) related to the objective level of fatigue from the two estimations of fatigue, a model of the unsupervised learning algorithm being chosen from the group consisting of: hierarchical grouping, partitioning into K-means, self-organizing maps, and Gaussian mixing.

9. The method (200) according to any of the preceding claims, wherein the biomathematics model is constructed from subjective and/or statistical data relating to a plurality of individuals.

10. The method (200) according to any of the preceding claims, wherein the operator's mission is the piloting of an aircraft and wherein the plurality of mission data comprise at least one type of data selected from the group comprising:
- data relating to the schedule of a crew of which the operator belongs;
- data relating to the configuration of an airline.

11. The method (200) of any preceding claim, wherein the physiological data comprises at least one type of data selected from the group consisting of:
- images of the operator;
- a heart rate;
- a blood pressure;
- oxygen respiration;
- sweating;
- oxygen saturation;
- a level of dehydration.

12. The method (200) according to any of the preceding claims, wherein the physiological data of the operator are measured during the mission.

13. A system of determination (16) of an objective level of fatigue comprising technical means configured to carry out the method according to any of the preceding claims.

14. A method of estimation and determination (100) of a level of fatigue of an operator, comprising the following steps:
- determination (110) of a first estimation of fatigue determined by a biomathematics model of prediction of fatigue from plurality of mission data related to the mission;
- determination (120) of a second estimation of fatigue determined by a physiological model of prediction of fatigue from physiological data of the operator; and
- implementation (130) of the determination method (200) according to one of claims 1 to 12.
